# EUROPÄISCHE PATENTSCHRIFT

(11) **EP 0 486 900 B1**
(45) Veröffentlichungstag und Bekanntmachung des Hinweises auf die Patenterteilung: **04.09.1996**
(21) Anmeldenummer: 91119053.6
(22) Anmeldetag: 08.11.1991
(51) Int. Cl.: C07D 209/08, C07D 209/86

(54) **Verfahren zur Herstellung von Indolen**
Process for the preparation of indoles
Procédé pour la préparation d'indoles

(30) Priorität: 21.11.1990 DE 4037004
(43) Veröffentlichungstag der Anmeldung: 27.05.1992
(73) Patentinhaber: BAYER AG, 51368 Leverkusen (DE)
(72) Erfinder: Blank, Heinz Ulrich, Dr., W-5068 Odenthal-Glöbuch (DE); Ullrich, Friedrich-Wilhelm, Dr., W-5000 Köln 80 (DE); Meisel, Karlheinrich, Dr., W-5068 Odenthal-Osenau (DE); Streicher, Willi, Dr., W-5000 Köln 80 (DE); Schulz, Nikolaus, Dr., W-5067 Kürten (DE); Irmscher, Dieter, Dipl.-Ing., W-5060 Bergisch Gladbach (DE); Klag, Günther, Dr., W-5090 Leverkusen (DE)

(56) Entgegenhaltungen:
- DE-A- 1 906 832
- DE-C- 574 840
- THE JOURNAL OF ORGANIC CHEMISTRY Bd. 33, Nr. 11, November 1968, COLUMBUS, OHIO, US Seiten 4283 - 4285; H. ILLY ET AL.: 'FISCHER INDOLE SYNTHESIS. DIRECTION OF CYCLIZATION OF ISOPROPYLMETHYL KETONE PHENYLHYDRAZONE'
- JOURNAL OF THE CHEMICAL SOCIETY 1950, LONDON Seiten 621 - 624; K.H. PAUSACKER: "THE FISCHER INDOLE SYNTHESIS. PART III. THE CYCLISATION OF THE PHENYLHYDRAZONES."
- R.K. BROWN IN THE CHEMISTRY OF HETEROCYCLIC COMPOUNDS: VOLUME 25, PART ONE: INDOLES, CHAPTER II 'THE SYNTHESIS OF THE INDOLE NUCLEUS' 1972 , JOHN WILEY & SONS, INC , NEW YORK

## Beschreibung

Die vorliegende Erfindung betrifft ein Verfahren zur Herstellung von Indolen durch Cyclisierung von Phenylhydrazonen, die aus Phenylhydrazin und Ketonen gebildet werden, in Gegenwart von weniger als 5 Äquivalenten Natriumhydrogensulfat.

Indole sind wichtige Zwischenprodukte in der Farbstoffindustrie. Es ist bekannt, Phenylhydrazone in organischen Medien unter Verwendung von Lewis- oder Protonensäuren zu cyclisieren, beispielsweise in Ethanol in Gegenwart von Zinkchlorid (Chem.-Zeitg. 22 (1898), 38), in Eisessig als Reaktionsmedium (J. Org. Chem. 42 (1977), 2474), in Chloroform in Gegenwart von Polyphosphorsäureester (Chem. and Ind. 1965, 473). Weiterhin ist eine solche Reaktion in Polyphosphorsäure ohne weiteren Lösungsmittelzusatz beschrieben (J. Am. Chem. Soc. 74 (1952), 3948).
Es ist weiterhin bekannt, diese Umsetzung in wäßrigem Medium mit Broensted-Säuren durchzuführen, wobei jedoch eine Säure mit einem pK-Wert unter 1,3 eingesetzt werden muß (DE-OS 19 06 832; J. Org. Chem. 33 (1968), 4283).

Es wurde nun überraschend gefunden, daß die Indolsynthese in wäßrigem Reaktionsmedium auch mit Natriumhydrogensulfat in kurzen Reaktionszeiten mit guten Ausbeuten durchgeführt werden kann, wobei weniger als 5 Äquivalente Natriumhydrogensulfat, bezogen auf das Phenylhydrazin, eingesetzt werden. Dies ist umso überraschender, als aus DE-OS 19 06 832 bekannt ist, daß bei Verwendung substituierter aliphatischer oder aromatischer Säuren, deren pK-Wert in der Nähe von 1,3 liegt, große Säuremengen und lange Reaktionszeiten erforderlich sind, um befriedigende Ausbeuten zu erzielen.

Es wurde ein Verfahren zur Herstellung von 1-H- bzw. 3-H-Indolen der Formel 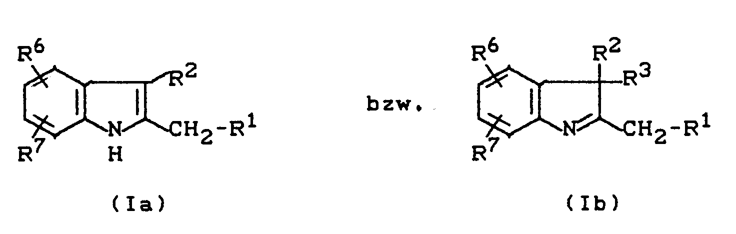 durch Umsetzung eines in mindestens einer ortho-Position nicht substituierten Phenylhydrazins der Formel 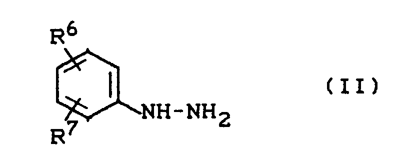 mit einem Keton der Formel 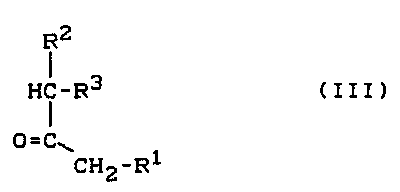 in wäßrigem Medium in Gegenwart einer sauren Verbindung gefunden, wobei in den Formeln
- R¹: für Wasserstoff steht,
- R²: Methyl bedeutet,
- R³: für Wasserstoff oder Methyl steht, wobei
- R¹: zusätzlich, wenn R³ Wasserstoff ist, für Methyl stehen kann,
- R¹ und R²: gemeinsam Trimethylen bedeuten können und
- R²: zusätzlich, wenn R¹ und R³ Wasserstoff sind, i-Propyl, n-Butyl oder n-Nonyl bedeuten kann,
- R⁶ und R⁷: unabhängig voneinander für Wasserstoff, geradkettiges oder verzweigtes C₁-C₁₂-Alkyl, geradkettiges oder verzweigtes C₁-C₄-Alkoxy, Phenyl, Phenoxy, Halogen, Hydroxy, Nitro, Cyano oder N(R⁸,R⁹) bedeuten, wobei R⁶ zusätzlich CON(R⁸,R⁹), COR⁸ oder COOR⁸ bedeuten kann und
- R⁸ und R⁹: unabhängig voneinander für Wasserstoff oder geradkettiges oder verzweigtes C₁-C₄-Alkyl stehen,
das dadurch gekennzeichnet ist, daß weniger als 5 Äquivalente, bezogen auf 1 Mol Phenylhydrazin, Natriumhydrogensulfat eingesetzt werden.

In bevorzugter Weise wird ein Phenylhydrazin mit mindestens einer nicht substituierten ortho-Position der Formel 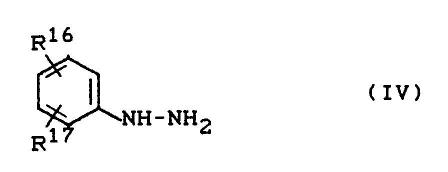 eingesetzt, worin
- R¹⁶ und R¹⁷: unabhängig voneinander Wasserstoff, geradkettiges oder verzweigtes C₁-C₁₂-Alkyl, geradkettiges oder verzweigtes C₁-C₄-Alkoxy, Phenyl, Phenoxy, Halogen, Hydroxy, Cyano oder N(R¹⁸,R¹⁹) bedeuten,
wobei R¹⁸ und R¹⁹ unabhängig voneinander für Wasserstoff, Methyl oder Ethyl stehen.

In besonders bevorzugter Weise wird ein Phenylhydrazin mit mindestens einer nicht substituierten ortho-Position der Formel 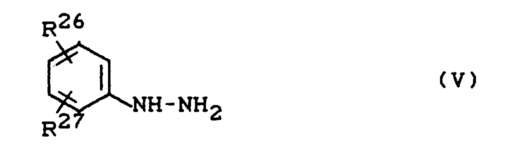 eingesetzt, worin
- R²⁶ und R²⁷: unabhängig voneinander Wasserstoff, Methyl, Ethyl, Methoxy, Ethoxy oder Halogen bedeuten und R²⁶ zusätzlich Hydroxy oder N(R¹⁸,R¹⁹) bedeuten kann.

Für den Fall, daß R³ Wasserstoff ist, bilden sich im erfindungsgemäßen Verfahren die 1-H-Indole; für den Fall, daß R³ nicht Wasserstoff ist, bilden sich die entsprechenden 3-H-Indole.

Geeignete Phenylhydrazine für das erfindungsgemäße Verfahren sind:
Phenylhydrazin, 4-Methylphenylhydrazin, 4-Dodecylphenylhydrazin, 2,5-Dimethylphenylhydrazin, 3,4-Dimethylphenylhydrazin, 4-Methoxyphenylhydrazin, 4-Hydroxyphenylhydrazin, 4-Chlorphenylhydrazin, 5-Chlor-2-methylphenylhydrazin, 2,4-Dichlorphenylhydrazin.

Geeignete Ketone für das erfindungsgemäße Verfahren sind:
2-Butanon, 3-Methyl-2-butanon, 3-Pentanon, 4-Methyl-2-pentanon, Cyclohexanon, 2-Methylcyclohexanon, 2-Dodecanon.

Es ist ein wesentliches Kennzeichen des erfindungsgemäßen Verfahrens, daß es in Gegenwart von Natriumhydrogensulfat durchgeführt wird, während die bisher bekannten Verfahren mit starken Säuren, beispielsweise Schwefelsäure oder Salzsäure, arbeiteten, die einen pK-Wert von zum Teil beträchtlich kleiner als 1,3 aufweisen.

Natriumhydrogensulfat wird in einer Menge von weniger als 5 Äquivalenten pro 1 Mol des eingesetzten Phenylhydrazins eingesetzt, bevorzugt in einer Menge von 1-4,9 Äquivalenten, besonders bevorzugt 1,5-4,5 Äquivalenten, ganz besonders bevorzugt 2-4 Äquivalenten.

Das erfindungsgemäße Verfahren wird bei einer Temperatur von 50-150°, bevorzugt 80-110°C durchgeführt.

Im erfindungsgemäßen Verfahren kann sowohl das Phenylhydrazin als auch das Keton jeweils gemeinsam mit Natriumhydrogensulfat im wäßrigen Medium vorgelegt werden, auf die Reaktionstemperatur gebracht werden und danach mit der noch fehlenden Umsetzungskomponente versetzt werden. Es ist häufig vorteilhaft, das Phenylhydrazin vorzulegen. Ebenso ist es möglich, das Phenylhydrazin und das Keton simultan in das auf Reaktionstemperatur befindliche wäßrige Medium mit Natriumhydrogensulfat zu geben; auch hierbei kann es vorteilhaft sein, etwas Phenylhydrazin vorzulegen und danach beide Komponenten simultan zuzugeben. Die simultane Zugabe eignet sich auch für eine kontinuierliche Arbeitsweise. In allen Fällen finden unmittelbar hintereinander die Bildung des Phenylhydrazons und dessen Cyclisierung zum Indol unter NH₃-Abspaltung statt.

Selbstverständlich kann auch ein separat hergestelltes Phenylhydrazon erfindungsgemäß umgesetzt werden.

Nach Beendigung des erfindungsgemäßen Verfahrens wird das Reaktionsgemisch neutralisiert. Hierbei fällt das Indol als organische Phase an; das Indol kann bei Bedarf in einer dem Fachmann geläufigen Weise weiter gereinigt werden. Die salzhaltige wäßrige Phase, die gleichzeitig bei der Neutralisation anfällt, wird in bekannter Weise entsorgt. Diese entsorgungsbedürftige Salzfracht ist im erfindungsgemäßen Verfahren kleiner als bei herkömmlichen Indolsynthesen.

Es ist eine besonders vorteilhafte Variante des erfindungsgemäßen Verfahrens, daß ein Phenylhydrazin eingesetzt wird, das aus dem zugehörigen Natriumphenylhydrazodisulfonat und/oder dem zugehörigen Natriumphenylhydrazo-β-sulfonat durch Zugabe von Schwefelsäure hergestellt wird. Diese Herstellung findet im allgemeinen in Gegenwart von restlichem Natriumsulfit und/oder Natriumhydrogensulfit statt, welches zur Bildung der Hydrazosulfonate eingesetzt worden war. In besonders vorteilhafter Weise wird für den Einsatz eines solchen Phenylhydrazins die benötigte Menge an Schwefelsäure so eingestellt, daß aus dem restlichen Natriumsulfit, Natriumhydrogensulfit und dem aus dem Hydrazosulfonat abgespaltenen Sulfat insgesamt weniger als 5 Äquivalente Natriumhydrogensulfat pro Mol Phenylhydrazin vorliegen.

In ein solches Phenylhydrazin, das die angegebene Menge an Natriumhydrogensulfat enthält, wird sodann das Keton gegeben, und das erfindungsgemäße Verfahren wird, wie oben näher beschrieben, durchgeführt.

In einer besonders bevorzugten Variante des erfindungsgemäßen Verfahrens wird auf die überraschende Möglichkeit zurückgegriffen, daß die Abspaltung von Sulfat aus dem Hydrazosulfonat bereits in Gegenwart des zur Indolsynthese benötigten Ketons vorgenommen werden kann. In diesem Falle findet in der selben Reaktionsmischung sowohl die Bildung des Phenylhydrazins als auch dessen weitere Umsetzung zum Indol statt.

Diese überraschende Erkenntnis wird in einer noch weiteren vorteilhaften Variante des erfindungsgemäßen Verfahrens soweit ausgedehnt, daß zur erfindungsgemäßen Herstellung des Indols von einem mindestens in einer ortho-Position nicht-substituierten Anilin der Formel 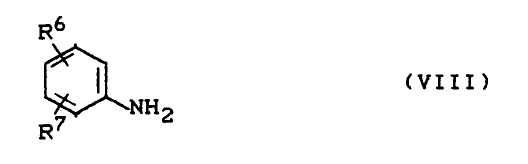 ausgegangen wird, worin R⁶ und R⁷ die oben angegebene Bedeutung haben, und die Reaktionsstufen des Diazotierens, der Reduktion des Diazoniumsalzes mit Sulfit, der Abspaltung der Sulfonatgruppe und die Endstufe der Umsetzung des so erhaltenen Phenylhydrazins mit dem Keton ohne Isolierung der Zwischenprodukte durchgeführt werden.

Diese geschilderten vorteilhaften Varianten des erfindungsgemäßen Verfahrens bringen eine weitere beträchtliche Reduzierung der Salzfracht mit sich. Ferner entfällt eine Belastung der Abwässer durch das nun nicht mehr zwischenisolierte Phenylhydrazin. Dies ist besonders bedeutungsvoll, da das Phenylhydrazin eine beträchtliche Wasserlöslichkeit aufweist, was einerseits zu einer schlechteren Gesamtausbeute bei herkömmlicher Zwischenisolierung führt und andererseits zu arbeitshygienischen Belastungen führt, da die Phenylhydrazine als krebserregend gelten. Wollte man die Wasserlöslichkeit des Phenylhydrazins drücken, um seine Verluste zu minimieren und die arbeitshygienischen Bedenken zu verringern, müßte man das Phenylhydrazin in Form seines Salzes mit einem großen Überschuß an Mineralsäure fällen, was ebenfalls zu einer Belastung des Abwassers führte. Diese geschilderten Probleme werden erfindungsgemäß überwunden.

### Beispiel 1

2 Mol Methylisopropylketon-phenylhydrazon wurden in eine 90°C warme Lösung von 5 Mol Natriumhydrogensulfat in 1900 ml Wasser in 30 min eingetropft. Die Mischung wurde 3 h bei 90 bis 100°C gerührt. Nach Abkühlen wurde der Ansatz neutralisiert, die organische Phase wurde abgetrennt und destilliert. Man erhielt 286,7 g 98,1 %iges 2,3,3-Trimethylindolenin. Ausbeute: 88,3 % der theoretischen Ausbeute.

### Beispiel 2

226 g (2 Mol) Phenylhydrazin (95,7 %ig) wurden bei 90°C in einer Lösung aus 5 Mol Natriumhydrogensulfat in 1900 ml Wasser vorgelegt. In 30 min wurden 225 ml (2,1 Mol) Methylisopropylketon zugetropft. Die Mischung wurde 3 h bei 90 bis 100° C gerührt. Nach Abkühlen wurde der Ansatz neutralisiert, die organische Phase wurde abgetrennt und destilliert. Man erhielt 299,3 g 97,5 %iges 2,3,3-Trimethylindolenin. Ausbeute: 91,6 % der theoretischen Ausbeute.

### Beispiel 3

Eine nach üblichen Methoden aus 2 Mol Anilin, 4,2 Mol Salzsäure und 2,02 Mol Natriumnitrit hergestellte wäßrige Benzoldiazoniumchloridlösung wurde nach bekannten Verfahren mit 5,2 Mol einer Mischung aus Natriumsulfit und Natriumbisulfit zu einer Phenylhydrazodisulfonatlösung umgesetzt. Bei 80° C wurden zu dieser Lösung 150 ml 48 %ige Schwefelsäure (1 Mol) getropft. 2 h wurde bei 80°C gerührt, dann wurden 225 ml (2,1 Mol) Methylisopropylketon in 30 min zudosiert. Nach 3 h Reaktionszeit bei 95 bis 100°C wurde abgekühlt. Der Ansatz wurde neutralisiert, die organische Phase wurde abgetrennt und destilliert. Man erhielt 276,3 g 98,6 %iges 2,3,3-Trimethylindolenin. Ausbeute: 85,5 % der theoretischen Ausbeute, bezogen auf Anilin.

### Beispiel 4

In gleicher Weise wie in Beispiel 3 wurde Anilin mit Methylethylketon umgesetzt. Es entstand 2,3-Dimethylindol mit einer Ausbeute von 93 % der theoretischen Ausbeute.

### Beispiel 5

In gleicher Weise wie in Beispiel 3 wurde Anilin mit Methylisobutylketon umgesetzt. Es entstand 3-Isopropyl-2-methylindol mit einer Ausbeute von 86 % der theoretischen Ausbeute.

### Beispiel 6

In gleicher Weise wie in Beispiel 3 wurde Anilin mit Cyclohexanon umgesetzt. Es entstand Tetrahydrocarbazol mit einer Ausbeute von 87 % der theoretischen Ausbeute.

### Beispiel 7

In gleicher Weise wie in Beispiel 3 wurde p-Toluidin mit Methylisopropylketon umgesetzt. Es entstand 2,3,3,5-Tetramethyl-3-H-indol mit einer Ausbeute von 87 % der theoretischen Ausbeute.

### Beispiel 8

In gleicher Weise wie in Beispiel 3 wurde p-Chloranilin mit Methylisopropylketon umgesetzt. Es entstand 2,3,3-Trimethyl-5-chlor-3-H-indol mit einer Ausbeute von 86 % der theoretischen Ausbeute.

### Beispiel 9

In einer kontinuierlichen Reaktion wurde Anilin mit Salzsäure und Natriumnitrit im Molverhältnis 1:2,3:1,07 diazotiert.

Diese Diazoniumsalzlösung wurde ebenfalls kontinuierlich mit 2,55 Mol Natriumsulfit/-bisulfit-Gemisch pro Mol Anilin zu einer Phenylhydrazodisulfonat-Lösung umgesetzt. Ein Anteil dieser Mischung, entsprechend 1,91 Mol Anilin, wurde bei 80°C zu 200 ml einer 48 %igen Schwefelsäure (1,35 Mol) getropft. Nach 2 h Reaktionszeit bei 80°C wurden 225 ml (2,1 Mol) Methylisopropylketon in 30 min zudosiert. Nach 3 h Reaktionszeit bei 95 bis 100°C wurde abgekühlt. Der Ansatz wurde neutralisiert, die organische Phase wurde abgetrennt und destilliert.

Man erhielt 273,8 g 98,2 %iges 2,3,3-Trimethylindolenin. Ausbeute: 88,4 % der theoretischen Ausbeute, bezogen auf Anilin.

### Beispiel 10

In gleicher Weise wie in Beispiel 2 wurde Phenylhydrazin mit 2-Heptanon umgesetzt. Es entstand 2-Methyl-3-butylindol mit einer Ausbeute von 38 %.

### Beispiel 11

27 g (0,25 Mol) Phenylhydrazin wurden bei 90°C in einer Lösung aus 1,12 Mol Natriumhydrogensulfat in 420 ml Wasser vorgelegt. In 30 min wurden 30 g (0,263 Mol) 2-Heptanon zudosiert. Die Mischung wurde 7 h bei 100° C gerührt. Nach Abkühlen wurde der Ansatz neutralisiert, die organische Phase wurde abgetrennt und destilliert. Man erhielt 2-Methyl-3-butylindol mit einer Ausbeute von 62 %.

## Patentansprüche

1. Verfahren zur Herstellung von 1-H- bzw. 3-H-Indolen der Formel 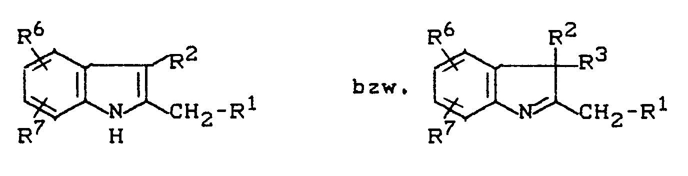 durch Umsetzung eines in mindestens einer ortho-Position nicht substituierten Phenylhydrazins der Formel 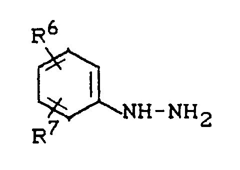 mit einem Keton der Formel 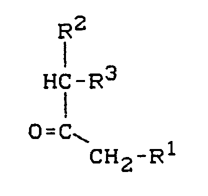 in wäßrigem Medium in Gegenwart einer sauren Verbindung, wobei in den Formeln
R¹ für Wasserstoff steht,
R² Methyl bedeutet,
R³ für Wasserstoff oder Methyl steht, wobei
R¹ zusätzlich, wenn R³ Wasserstoff ist, für Methyl stehen kann,
R¹ und R² gemeinsam Trimethylen bedeuten können und
R² zusätzlich, wenn R¹ und R³ Wasserstoff sind: i-Propyl, n-Butyl oder n-Nonyl bedeuten kann,
R⁶ und R⁷ unabhängig voneinander Wasserstoff, geradkettiges oder verzweigtes C₁-C₁₂-Alkyl, geradkettiges oder verzweigtes C₁-C₄-Alkoxy, Phenyl, Phenoxy, Halogen, Hydroxy, Nitro, Cyano oder N(R⁸,R⁹) bedeuten, wobei R⁶ zusätzlich CON(R⁸,R⁹), COR⁸ oder COOR⁸ bedeuten kann, und
R⁸ und R⁹ unabhängig voneinander für Wasserstoff oder geradkettiges oder verzweigtes C₁-C₄-Alkyl stehen,
dadurch gekennzeichnet, daß weniger als 5 Äquivalente, bezogen auf 1 Mol Phenylhydrazin, Natriumhydrogensulfat eingesetzt werden.

2. Verfahren nach Anspruch 1, dadurch gekennzeichnet, daß ein Phenylhydrazin mit mindestens einer nicht substituierten ortho-Position der Formel 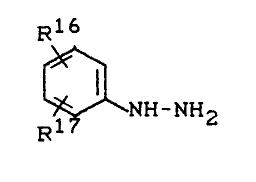 eingesetzt wird, worin
R¹⁶ und R¹⁷ unabhängig voneinander Wasserstoff, geradkettiges oder verzweigtes C₁-C₁₂-Alkyl, geradkettiges oder verzweigtes C₁-C₄-Alkoxy, Phenyl, Phenoxy, Halogen, Hydroxy, Cyano oder N(R¹⁸,R¹⁹) bedeuten,
wobei R¹⁸ und R¹⁹ unabhängig voneinander für Wasserstoff, Methyl oder Ethyl stehen,
daß bevorzugt ein Phenylhydrazin mit mindestens einer nicht-substituierten ortho-Position der Formel 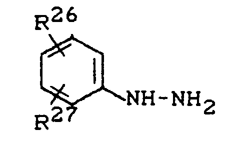 eingesetzt wird, worin
R²⁶ und R²⁷ unabhängig voneinander Wasserstoff, Methyl, Ethyl, Methoxy, Ethoxy oder Halogen bedeuten und R²⁶ zusätzlich Hydroxy oder N(R¹⁸,R¹⁹) bedeuten kann.

3. Verfahren nach Anspruch 1, dadurch gekennzeichnet, daß 1-4,9 Äquivalente, bevorzugt 1,5-4,5 Äquivalente, besonders bevorzugt 2-4 Äquivalente Natriumhydrogensulfat pro 1 Mol Phenylhydrazin eingesetzt werden.

4. Verfahren nach Anspruch 1, dadurch gekennzeichnet, daß es bei 50-150°C, bevorzugt 80-110°C durchgeführt wird.

5. Verfahren nach Anspruch 1, dadurch gekennzeichnet, daß ein Phenylhydrazin eingesetzt wird, das aus dem zugehörigen Alkaliphenylhydrazodisulfonat und/oder dem zugehörigen Alkaliphenylhydrazo-β-sulfonat in Gegenwart von restlichem Natriumsulfit und/oder Natriumhydrogensulfit durch Zugabe von Schwefelsäure hergestellt wird.

6. Verfahren nach Anspruch 5, dadurch gekennzeichnet, daß die Menge Schwefelsäure so eingestellt wird, daß aus dem restlichen Natriumsulfit, Natriumhydrogensulfit und dem aus dem Hydrazosulfonat abgespaltenen Sulfat insgesamt weniger als 5 Äquivalente Natriumhydrogensulfat pro Mol Hydrazin vorliegen.

7. Verfahren nach Anspruch 5, dadurch gekennzeichnet, daß die Abspaltung der Sulfonatgruppe aus dem Natriumphenylhydrazodisulfonat und/oder dem Natriumphenylhydrazo-β-sulfonat in Gegenwart des Ketons vorgenommen wird und die Indolsynthese ohne Zwischenisolierung des Phenylhydrazins in diesem Medium vorgenommen wird.

8. Verfahren nach Anspruch 7, dadurch gekennzeichnet, daß zur Herstellung des Indols von einem mindestens in einer ortho-Position nicht-substituierten Anilin der Formel 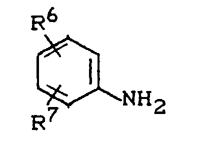 ausgegangen wird, worin
R⁶ und R⁷ unabhängig voneinander Wasserstoff, geradkettiges oder verzweigtes C₁-C₁₂-Alkyl, geradkettiges oder verzweigtes C₁-C₄-Alkoxy, Phenyl, Phenoxy, Halogen, Hydroxy, Nitro, Cyano oder N(R⁸,R⁹) bedeuten, wobei R⁶ zusätzlich CON(R⁸,R⁹), COR⁸ oder COOR⁸ bedeuten kann,
und die Reaktionsstufen des Diazotierens, der Reduktion des Diazoniumsalzes mit Sulfit, der Abspaltung der Sulfonatgruppe und die Endstufe der Umsetzung mit dem Keton ohne Isolierung der Zwischenprodukte durchgeführt wird.

## Claims

1. Process for the preparation of 1-H- or 3-H-indoles of the formula 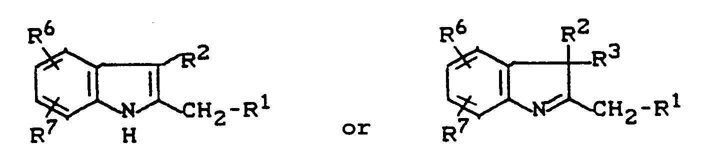 by reaction of a phenylhydrazine of the formula 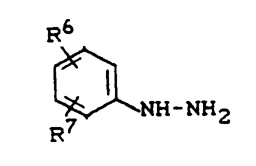 which is unsubstituted in at least one ortho-position, with a ketone of the formula 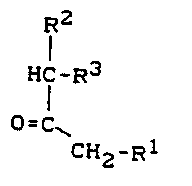 in an aqueous medium in the presence of an acid compound, where, in the formulae,
R¹ represents hydrogen,
R² denotes methyl,
R³ represents hydrogen or methyl, it being possible for
R¹ additionally to represent methyl, if R³ is hydrogen,
R¹ and R² together to denote trimethylene and
R² additionally to denote i-propyl, n-butyl or n-nonyl, if R¹ and R³ are hydrogen,
R⁶ and R⁷ independently of one another represent hydrogen, straight-chain or branched C₁-C₁₂-alkyl, straight-chain or branched C₁-C₄-alkoxy, phenyl, phenoxy, halogen, hydroxyl, nitro, cyano or N(R⁸,R⁹),
it being possible for R⁶ additionally to denote CON(R⁸,R⁹), COR⁸ or COOR⁸, and
R⁸ and R⁹ independently of one another represent hydrogen or straight-chain or branched C₁-C₄-alkyl,
characterised in that less than 5 equivalents, based on 1 mol of phenylhydrazine, of sodium hydrogen sulphate are employed.

2. Process according to Claim 1, characterised in that a phenylhydrazine having at least one unsubstituted ortho-position, of the formula 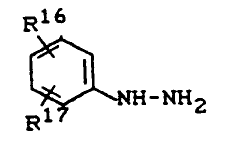 is employed, wherein
R¹⁶ and R¹⁷ independently of one another denote hydrogen, straight-chain or branched C₁-C₁₂-alkyl, straight-chain or branched C₁-C₄-alkoxy, phenyl, phenoxy, halogen, hydroxyl, cyano or N(R¹⁸,R¹⁹),
R¹⁸ and R¹⁹ independently of one another representing hydrogen, methyl or ethyl,
and in that a phenylhydrazine having at least one unsubstituted ortho-position, of the formula 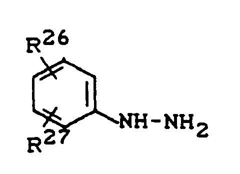 is preferably employed, wherein
R²⁶ and R²⁷ independently of one another denote hydrogen, methyl, ethyl, methoxy, ethoxy or halogen and R²⁶ additionally can denote hydroxyl or N(R¹⁸,R¹⁹).

3. Process according to Claim 1, characterised in that 1-4.9 equivalents, preferably 1.5-4.5 equivalents and particularly preferentially 2-4 equivalents of sodium hydrogen sulphate are employed per 1 mol of phenylhydrazine.

4. Process according to Claim 1, characterised in that it is carried out at 50-150°C, preferably 80-110°C.

5. Process according to Claim 1, characterised in that a phenylhydrazine is employed which is prepared from the associated alkali metal phenylhydrazodisulphonate and/or the associated alkali metal phenylhydrazo-β-sulphonate in the presence of residual sodium sulphite and/or sodium hydrogen sulphite by addition of sulphuric acid.

6. Process according to Claim 5, characterised in that the amount of sulphuric acid is adjusted such that, from the residual sodium sulphite, sodium hydrogen sulphite and the sulphate eliminated from the hydrazosulphonate, in total less than 5 equivalents of sodium hydrogen sulphate are present per mole of hydrazine.

7. Process according to Claim 5, characterised in that the elimination of the sulphonate group from the sodium phenylhydrazodisulphonate and/or the sodium phenylhydrazo-β-sulphonate is carried out in the presence of the ketone and the indole synthesis is carried out without intermediate isolation of the phenylhydrazine in this medium.

8. Process according to Claim 7, characterised in that an aniline the formula 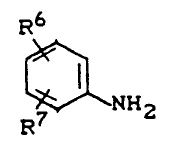 wherein
R⁶ and R⁷ independently of one another denote hydrogen, straight-chain or branched C₁-C₁₂-alkyl, straight-chain or branched C₁-C₄-alkoxy, phenyl, phenoxy, halogen, hydroxyl, nitro, cyano or N(R⁸,R⁹), it being possible for R⁶ additionally to denote CON(R⁸,R⁹), COR⁸ or COOR⁸,
is used as the starting material for the preparation of the indole and the reaction steps of diazotisation, reduction of the diazonium salt by means of sulphite, elimination of the sulphonate group and the final step of the reaction with the ketone are carried out without isolation of the intermediates.

## Revendications

1. Procédé de préparation des 1-H- et 3-H-indoles de formules respectives 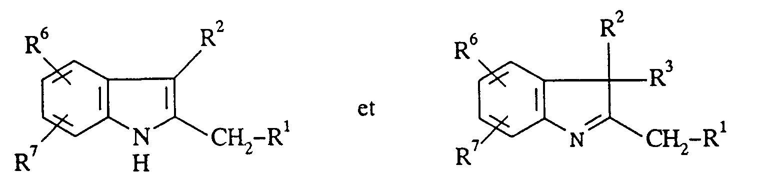 par réaction d'une phénylhydrazine non substituée dans au moins une position ortho et répondant à la formule 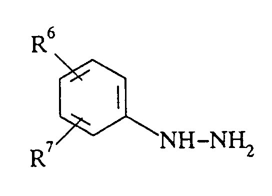 avec une cétone de formule 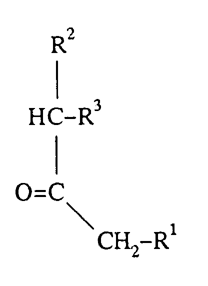 en milieu aqueux, en présence d'un composé acide, les symboles des formules ci-dessus ayant les significations suivantes :
R¹ représente l'hydrogène,
R² représente un groupe méthyle,
R³ représente l'hydrogène ou un groupe méthyle,
R¹ pouvant en outre représenter un groupe méthyle lorsque R³ représente l'hydrogène,
R¹ et R² peuvent également former ensemble un groupe triméthylène et
R² peut en outre représenter un groupe isopropyle, n-butyle ou n-nonyle lorsque
R¹ et R³ représentent l'hydrogène,
R⁶ et R⁷ représentent chacun, indépendamment l'un de l'autre, l'hydrogène, un groupe alkyle à chaîne droite ou ramifiée en C₁-C₁₂, un groupe alcoxy à chaîne droite ou ramifiée en C₁-C₄, un groupe phényle, phénoxy, un halogène, un groupe hydroxy, nitro, cyano ou N(R⁸,R⁹),
R⁶ pouvant en outre représenter CON(R⁸R⁹), COR⁸ ou COOR⁸ et
R⁸ et R⁹ représentent chacun, indépendamment l'un de l'autre, l'hydrogène ou un groupe alkyle à chaîne droite ou ramifiée en C₁-C₄,
caractérisé en ce que l'on met en oeuvre du bisulfate de sodium en quantité inférieure à cinq équivalents pour une mole de phénylhydrazine.

2. Procédé selon la revendication 1, caractérisé en ce que l'on met en oeuvre une phénylhydrazine ayant au moins une position ortho non substituée et répondant à la formule 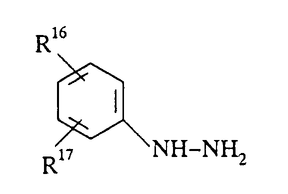 dans laquelle
R¹⁶ et R¹⁷ représentent chacun, indépendamment l'un de l'autre, l'hydrogène, un groupe alkyle à chaîne droite ou ramifiée en C₁-C₁₂, un groupe alcoxy à chaîne droite ou ramifiée en C₁-C₄, un groupe phényle, phénoxy, un halogène, un groupe hydroxy, cyano ou N(R¹⁸,R¹⁹),
R¹⁸ et R¹⁹ représentant chacun, indépendamment l'un de l'autre, l'hydrogène, un groupe méthyle ou éthyle,
de préférence une phénylhydrazine ayant au moins une position ortho non substituée et répondant à la formule 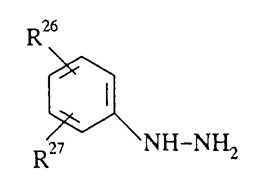 dans laquelle
R²⁶ et R²⁷ représentent chacun, indépendamment l'un de l'autre, l'hydrogène, un groupe méthyle, éthyle, méthoxy, éthoxy ou un halogène, R²⁶ pouvant en outre représenter un groupe hydroxy ou N(R¹⁸,R¹⁹).

3. Procédé selon la revendication 1, caractérisé en ce que l'on met en oeuvre de 1 à 4,9 équivalents, de préférence de 1,5 à 4,5 équivalents et tout spécialement de 2 à 4 équivalents de bisulfate de sodium par mole de phénylhydrazine.

4. Procédé selon la revendication 1, caractérisé en ce que l'on opère à des températures de 50 à 150°C, de préférence de 80 à 110°C.

5. Procédé selon la revendication 1, caractérisé en ce que l'on met en oeuvre une phénylhydrazine qui a été préparée à partir du phénylhydrazodisulfonate alcalin correspondant et/ou du phénylhydrazo-β-sulfonate alcalin correspondant en présence du sulfite de sodium et/ou du bisulfite de sodium résiduels par addition d'acide sulfurique.

6. Procédé selon la revendication 5, caractérisé en ce que l'on règle la quantité d'acide sulfurique en sorte que le sulfate libéré à partir du sulfite et du bisulfite de sodium résiduel et de l'hydrazosulfonate, représente moins de 5 équivalents de bisulfate de sodium par mole d'hydrazine.

7. Procédé selon la revendication 5, caractérisé en ce que la scission du groupe sulfonate à partir du phénylhydrazodisulfonate de sodium et/ou du phénylhydrazo-β-sulfonate de sodium est réalisée en présence de la cétone et en ce que la synthèse de l'indole est réalisée dans le même milieu sans isolement préalable de la phénylhydrazine.

8. Procédé selon la revendication 7, caractérisé en ce que, pour la préparation de l'indole, on part d'une aniline non substituée dans au moins une position ortho et répondant à la formule 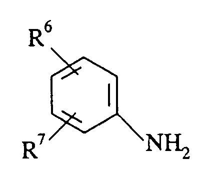 dans laquelle R⁶ et R⁷ représentent chacun, indépendamment l'un de l'autre, l'hydrogène, un groupe alkyle à chaîne droite ou ramifiée en C₁-C₁₂, un groupe alcoxy à chaîne droite ou ramifiée en C₁-C₄, un groupe phényle, phénoxy, un halogène, un groupe hydroxy, nitro, cyano ou N(R⁸,R⁹),
R⁶ pouvant en outre représenter CON(R⁸,R⁹), COR⁸ ou COOR⁸, et on procède aux opérations de diazotation, de réduction du sel de diazonium à l'aide d'un sulfite, de scission du groupe sulfonate et finalement de réaction avec la cétone sans isoler les produits intermédiaires.
